# EUROPEAN PATENT SPECIFICATION

(11) **EP 2 842 602 B1**
(45) Date of publication and mention of the grant of the patent: **27.06.2018**
(21) Application number: 14156468.2
(22) Date of filing: 21.12.2011
(51) Int. Cl.: A61N 5/10, A61N 5/06

(54) **A mobile X-ray unit**
Mobile Röntgeneinheit
Unité à rayons X mobile

(30) Priority: 22.12.2010 NL 2005906; 23.12.2010 US 201061426896 P
(43) Date of publication of application: 04.03.2015
(62) Divisional of application: 11811415.6
(73) Proprietor: Nucletron Operations B.V., 3905 TH Veenendaal (NL)
(72) Inventor: Woudstra, Bas, 3905 TH Veenendaal (NL); de Jager, Wim, 3905 TH Veenendaal (NL)
(74) Representative: V.O.

(56) References cited:
- WO-A2-2008/118198
- US-A1- 2003 048 875
- US-A1- 2005 276 377
- Topex, Inc: "SRT 100 Superficial Radiotherapy System for the treatment of Skin Cancer", , 31 December 2007 (2007-12-31), XP002656846, Retrieved from the Internet: URL:http://www.harpell.ca/wp-content/uploa ds/2009/11/topexbrochure_v10.pdf [retrieved on 2011-08-25]
- Topex, Inc: "Regulatory Information", , 31 December 2007 (2007-12-31), XP002656847, Retrieved from the Internet: URL:http://www.topexmedical.com/product2.h tml [retrieved on 2011-08-25]

## Description

### FIELD OF THE INVENTION

The invention relates to a mobile X-ray unit comprising an applicator cap as set out in claim 1.

### BACKGROUND OF THE INVENTION

Skin cancer, having increased incidence rate in the last decade of the 20^{th} century, requires substantial effort from medical professionals in terms of early diagnosis, logistics and availability of suitable treatment. However, it is appreciated that over 1.3 million new skin cancers are diagnosed annually and are increasing at a rate of about 5 % per year. Increased exposure to the sun without skin protection and a decreased ozone layer are regarded as the main causes of this increase - a problem estimated to be costing over 1 billion Euros in annual medical treatment expenses. Over 80% of skin cancers occur in the head and neck regions with 50% occurring in patients over 60 years of age. It is expected that a portion of the senior population will double in year 2025 compared to the present demographics.

Non proliferated cancers being substantially superficial lesions may be treated in different ways. First, surgery may be envisaged. However, such technique may be disadvantageous in terms of long waiting lists and complications related to post-treatment care. In addition, due to invasive character of surgery contamination of the wound by infections may present an additional risk. Secondly, irradiation using electrons of soft X-rays may be envisaged. Such techniques have an advantage of being non invasive, wherein a treatment session may be as short as 2 to 4 minutes. It will be appreciated that usually the integral treatment using radiotherapeutic techniques may comprise a number of sessions.

Accordingly, the growing incidence of skin cancer and increasing of a share of the senior population in overall demographics pose substantial challenge on the cancer treatment logistics.

Recently, the use of a mobile and portable X-ray unit has been suggested, which may be used inside a hospital radiotherapy department. An embodiment of such portable unit is described in US 2007/0076851. The known unit comprises an X-ray source provided with a filtering device having a plurality of filters rotatably arranged with respect to a focal point of the X-ray tube for changing filtering characteristics on demand. The plurality of filters is arranged in a filtering device, which is transversely arranged with respect to a longitudinal axis of the X-ray tube.

It is a disadvantage of the known X-ray tube that beam characteristics due to internal geometry of the known X-ray tube may be detrimentally affected, for example leading to a broadened penumbra of the X-ray beam.

Another mobile X-ray unit is the "SRT 100" from TOPEX.

### SUMMARY OF THE INVENTION

It is an object of the invention to provide a mobile X-ray unit having improved operational characteristics. In particular, it is an object of the invention to provide a mobile X-ray unit intercepting secondary electrons emanating from the X-ray applicator.

To this end, the mobile X-ray unit according to the invention is provided with an applicator cap manufactured from polyphenylsulfone (0.3mm-0.6mm thick) or polyvinylidene fluoride (0.4mm-0.7mm thick).

Also, the mobile X-ray unit may have an improved penumbra of the X-ray beam and/or a reduced skin dose, when dose delivery is specified at 5 mm depth.

To this end in the mobile X-ray unit a distance between the target and the collimator may be in the range between 4 and 10 cm.

It will be appreciated that the terms 'mobile' and 'portable' in the context of the present application may be interchanged as these terms equally relate to an easily moved or transported device, for example, a device which may be moved or transported by a single individual.

It is found that by setting a distance between the X-ray target and the collimator in the range of 4...10 cm, preferably to a distance of about 5 to 6 cm improved beam characteristics are achieved. For example, it is found that improved beam flatness as well as sharpened penumbra are achievable for the target-collimator distance of 4...10 cm, particularly for the target-collimator distance of about 5 to 6 cm due to a relatively small focal size. For example, for the target-collimator distance of about 5 cm penumbra of 1.5 - 1.8 mm is achievable (specified for 20/80% lines).

It is appreciated that such sharpened penumbra is important particularly for treating of small lesions, like skin cancers, as dose to healthy tissue, being a critical item in dose delivery planning, is minimized.

In an example of an X-ray unit, the target and the collimator are accommodated in a substantially cylindrically shaped X-ray tube having a longitudinal axis, a direction of propagation of the X-ray beam being substantially parallel to said longitudinal axis.

It is found to be advantageous to arrange the anode-collimator geometry in such a way that the axis of the X-ray tube substantially coincides with a direction of propagation of the generated X-ray beam. Thus, the X-ray tube and the X-ray applicator may have the same longitudinal axis. Such configuration is advantageous from mechanical perspective as balancing of the applicator on the articulated arm is simplified for a coaxial geometry. It will be appreciated that the X-ray tube, accommodated in the X-ray applicator represents a relatively slim (outer diameter of less than 10 cm) elongated cylinder (length of about 30 cm), which is preferably displaced in a vertical direction for delivering the X-ray beam to the patient. Once the internal geometry of the X-ray tube is coaxial, the weight of the X-ray tube may be suitably balanced enabling easy and reproducible displacement of the articulated arm supporting the X-ray applicator.

In a further example of the X-ray unit, the collimator is provided with automatic identification means arranged to generate a signal in the control unit representative of collimator characteristics.

It is found to be advantageous to enable a fully automatic identification of the collimator inserted in the X-ray tube, as human errors with respect to defining the field geometry may be minimized or even eliminated. For example, in case when the collimator is conceived to be provided in a receptacle, such receptacle may be provided with a resistive path whose resistivity may be changed. The collimator may then be arranged with projections adapted to cooperate with the resistive path of the receptacle for changing the resulting resistivity and thus for generating a signal representative of the collimator being inserted. Preferably, the signal is made available to the control unit of the mobile X-ray unit for independent verification. Preferably, the X-ray unit comprises a set of collimators provided with respective identification means.

In a still further example of the X-ray unit, it is provided with a signaling means indicating generation of the X-ray beam.

It is found advantageous to provide means of signaling that the X-ray beam is on. For example, such signaling may be implemented as a suitable light on the X-ray applicator. One or more light emitting diodes may be used for this purpose. It may be possible to provide a plurality of signaling means in dependence of the energy of the generated X-ray beam.

For example, for the X-ray beam of a lower portion of the spectrum (about 50 kV) a first indicator may be used, for example a first light color. For an intermediate portion of the spectrum (about 60 - 65 kV) a second indicator may be used, for example a second light color. Finally, for the higher portion of the spectrum (66 - 75 kV, preferably 66 - 70 kV) a third indicator may be used, for example a third light color. It will be appreciated that a plurality of possibilities exist for indicating different spectra, including but not limited to a progressive illumination of a plurality of indicators upon hardening of the delivered X-ray beam. It will be further appreciated that such indication of the kV range may be allowed in the device, in a user interface or in a supplementary unit. It will be further appreciated that the named kV ranges may be scaled, for example with the factors 1,1; 1,2; 1,3; 1,4; 1,5. Preferably, the signaling means comprises a light indicator arranged on the outer housing. Such arrangement of the signaling means is advantageous as the patient is made aware about the starting point and the termination of irradiation so that the patient may retain a static position during the course of treatment.

In a further example of an X-ray unit the cooler is arranged with piping to provide a cooling medium in a vicinity of the X-ray tube, the piping running in a space between the X-ray tube and a shielding wall associated with the X-ray tube.

It is found to be advantageous to provide a spacing between the outer surface of the X-ray tube and the inner surface of the X-ray tube, said spacing being at least partially filled with a coolant. It is found to be advantageous to provide circulated water as a cooling agent due to high specific heat capacity, offering improved heat transfer of water with respect to a gas. However, pressurized gas may also be used as a suitable coolant. Preferably, a temperature sensor is arranged on the outer housing of the X-ray applicator for measuring actual temperature of the outer housing. The temperature sensor may be connected to the control unit for controlling the cooler and/or for controlling the high voltage supply. Should temperature rise above a pre-determined shut-off value, the control unit may be arranged to disable the high voltage supply and/or to intensify the cooling mode, for example by increasing a pumping capacity of the coolant.

In a still further example of the X-ray unit, a radiation detector is provided inside the outer housing for detecting the X-ray beam.

It is found to be advantageous to provide independent means for detecting presence of the generated X-ray beam. Preferably, the X-ray unit comprises a primary timer which sets a time for the high voltage supply for delivering a predetermined radiation dose. The radiation sensor accommodated inside the outer housing of the X-ray applicator may be part of a secondary timer circuit adapted to shut down the high voltage supply upon the event the predetermined radiation dose is delivered. In this way radiation safety control may be improved.

In the X-ray unit according to the invention, the X-ray applicator comprises an exit surface conceived to be directed towards a patient, said surface being covered by an applicator cap. It is found advantageous to provide such applicator cap, which may have many functions in use. First, the applicator cap may be used for protecting the exit surface of the X-ray applicator from intra-patient contamination. Secondly, thickness of the cap in a direction of the beam propagation may be selected to be sufficient for substantially eliminating electron contamination from the X-ray beam. It will be appreciated that those skilled in the art will readily appreciate the relation between the energy of the secondary electrons emanating from the X-ray tube and a required thickness of a given material, for example plastic, glass, ceramics sufficient for fully intercepting these electrons. Preferably, the applicator cap is disposable.

Thirdly, the applicator cap may function as a heat absorber for mitigating the elevated temperature of the X-ray applicator in use. As a result the patient will feel the applicator contacting the skin as a slightly warm object.

In a still further example of the X-ray unit, the X-ray applicator is connected to the base using a displaceable panel, the flexible cabling running substantially in the displaceable panel.

It is found to be advantageous to provide an intermediate mechanical unit connecting the base of the mobile X-ray unit and the X-ray applicator for housing the flexible cables thereby preventive their entanglement. The displaceable panel may be arranged with a pre-defined travel distance with respect to a lowest achievable stand and a highest achievable stand. Such predefined travel distance may be advantageous for increasing durability of the cables tubes and wiring of the X-ray unit, especially of the tubes accommodating the coolant.

In a still further example of the X-ray unit, the displaceable panel comprises a user interface for controlling the X-ray unit. Preferably, the user interface comprises a display. For example, the display may be implemented as a touch screen arranged for enabling data input. Alternatively, display may be arranged for echoing data, whereas dedicated buttons or other suitable means may be provided for entering input data into the X-ray unit. Also, there is provided a method for manufacturing a mobile X-ray unit comprising a base for accommodating a control unit, a power supply and a cooler and further comprising an articulated displaceable X-ray arm supporting an X-ray applicator comprising an X-ray tube, comprising the steps of:
- connecting said arm to the base using a flexible cable;
- arranging the X-ray tube with a target for generating an X-ray beam and a collimator for shaping the generated X-ray beam;
- setting a distance between the target and the collimator in the range between 4 and 10 cm.

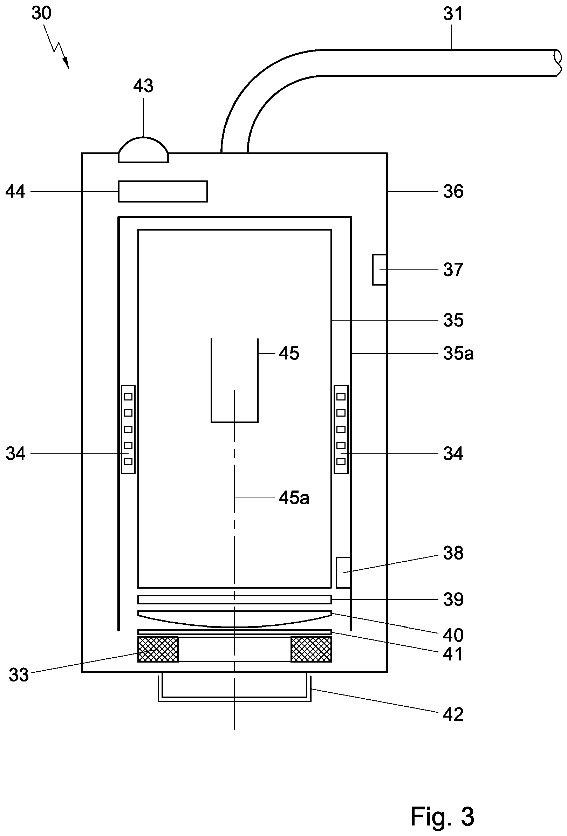

Preferably, in the X-ray unit, the target and the collimator are accommodated in a substantially cylindrically shaped X-ray applicator having a longitudinal axis, a direction of propagation of the X-ray beam being substantially parallel to said longitudinal axis. Further advantageous examples of the method will be discussed with reference to Figure 3.

In a method of delivering an X-ray beam for irradiating a superficial lesion, wherein an X-ray unit comprises a base for accommodating a control unit, a power supply and a cooler and further comprising an articulated displaceable X-ray arm accommodating an X-ray tube, said arm being connected to the base using a flexible cable, the X-ray tube comprises a target for generating an X-ray beam and a collimator for shaping the generated X-ray beam, a distance between the target and the collimator being in the range between 4 and 10 cm.

The invention relates to a mobile X-ray unit with an applicator cap for an X-ray unit comprising an X-ray tube accommodated in an X-ray applicator, said X-ray applicator comprising an exit surface conceived to be directed towards a patient, the applicator cap being arranged for covering at least said surface. Preferably, the applicator cap is disposable. More preferably, thickness of the cap in a direction of the beam propagation is sufficient for substantially eliminating electron contamination from the X-ray beam. An applicator cap may be advantageously manufactured from a substantially transparent material for enabling visualization of delineation between the exit surface of the X-ray applicator and a lesion conceived to be treated. Also, a mobile medical care unit may be provided, such as a bed, a chair, a trolley, a cart, a galley or a treatment unit, comprising at three, four or more wheels, wherein at least some wheels are interconnected by a flexible frame allowing automatic adjustment of the height of the wheel when contacting a ground surface.

For example, the frame may comprise one or more branches working together or individually. The said one or more branches may be provided with a weak region allowing the branch to be deformable by application of the weight of the mobile medical care unit when being moved over the ground.

More in particular, the frame may comprise flexible regions, adapted to be resilient and/or bendable under application of the weight of the medical care unit. In a particular embodiment of the mobile medical care device the flexible frame comprises one or more branches, one or each of them is built from one or more segments coupled by a spring.

It is found that such flexible frame has a particular advantage when the mobile medical care unit is transported over an uneven floor, or a floor having irregularities, such as bumps.

It will be appreciated that for many applications, it may be desirable that the mobile medical care unit does not change its spatial orientation even when being transported over an irregular surface. For example, laboratory trays, beds, in particular neonatal beds, food supply trays and so on are preferably kept in a substantially constant orientation when transported.

More in particular, the mobile X-ray device according to the foregoing has an advantage when the base is provided with wheels which are supported by a flexible frame. One of particular modes of application of the mobile X-ray device is a mobile clinic, that is when the mobile X-ray device is provided within a vehicle and is transported to different treatment locations. In particular circumstances the treatment may be carried out in inferior conditions, even treatment in open air is possible. By providing the mobile X-ray unit with a possibility of self-adaptation to the surface irregularities, the adjustment of the X-ray applicator may be carried out in substantially the same way as if the treatment is carried out in a doctor's office. In addition, by ensuring that the X-ray applicator is located in substantially in the same orientation when stored, the doctor would need to go through substantially the same positioning routine when locating the X-ray applicator for treatment. Accordingly, human errors due to a complex three-dimensional handling of the X-ray applicator may be avoided.

These and other aspects of the invention will be discussed with reference to drawings wherein like reference numerals or signs relate to like elements. It will be appreciated that the drawings are presented for illustration purposes only and may not be used for limiting the scope of the appended claims.

### BRIEF DESCRIPTION OF THE DRAWINGS

Figure 1a presents in a schematic way an example of a mobile X-ray unit.
Figure 1b presents in a schematic way an example of a displaceable panel of the mobile X-ray unit.
Figure 1c presents in a schematic way an example of displacement functionality of the applicator of the X-ray unit.
Figure 2 presents in a schematic way an example of architecture of the mobile X-ray unit.
Figure 3 presents in a schematic way an example of a cross section of an X-ray applicator of the mobile X-ray unit.
Figure 4 presents in a schematic way an example of the X-ray applicator of Figure 3 provided with an applicator cap.
Figure 5 presents in a schematic way an example of a collimator provided with identification means.
Figure 6 presents in a schematic view an embodiment of a collimator identification system.
Figure 7 presents in a schematic view the X-ray tube according to the invention.
Figure 8 presents in a schematic way an example of a medical care unit, such as a mobile X-ray unit.
Figure 9 presents a further view of the connection between the various components of the flexible frame.
Figure 10 presents a further schematic view of an example shown in Figure 9.

### DETAILED DESCRIPTION OF THE DRAWINGS

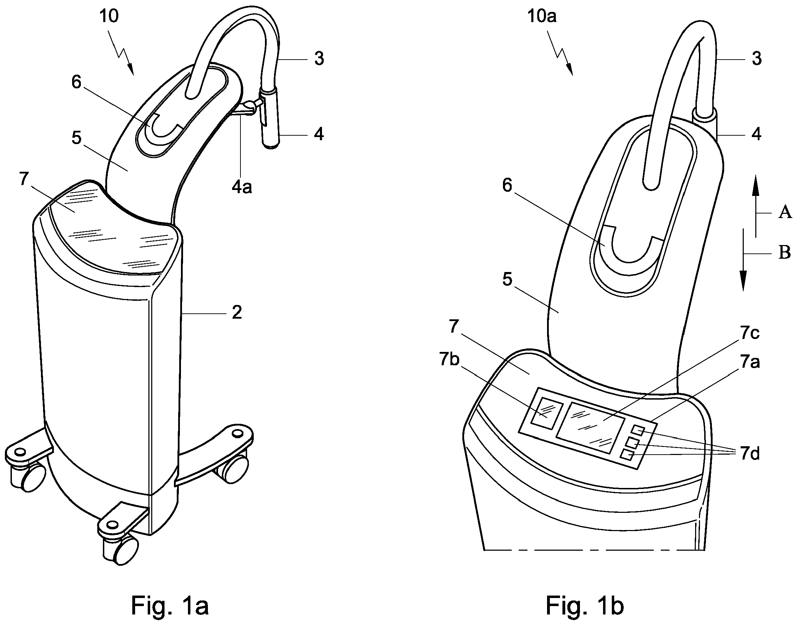

Figure 1a presents in a schematic way an example of a mobile X-ray unit. The mobile X-ray unit 10 comprises a base 2 comprising at least a power supply unit, a cooling system and a control unit for controlling an operation of the X-ray applicator 4 comprising an X-ray tube accommodated in an outer housing. The X-ray applicator 4 is connected with the base using flexible cables 3, which may be at least partially received in a displaceable panel 5. The applicator 4 is supported by an articulated displaceable arm 4a, which may comprise a pivot for altering angulation of the applicator 4 in space. The articulated arm 4a may also be mechanically connected with the displaceable panel 5 for enabling alteration of a vertical position of the applicator 4. Preferably, the displaceable panel 5 is provided with a handle 6 enabling easy manipulation thereof. The displaceable panel 5 may be guided along suitable rails for enabling a substantially smooth and shock-free displacement thereof.

The displaceable panel 5 may be also referred to as a displaceable mast. It is found to be advantageous to allow the mast to be displaceable along a substantially upright axis with respect to the base 2. It will be appreciated that the substantially upright axis extends in a substantially vertical direction, which is generally upright. However, it will be further appreciated that the terms 'generally upright' or 'substantially vertical' may relate to a direction substantially perpendicular (+- 20 degrees) to a plane of the surface on which the mobile X-ray unit is sitting.

Preferably, the base 2 is provided with a display 7 for feeding-back suitable user information. The display 7 may be arranged as a touch-sensitive screen for enabling suitable data input into the system.

Figure 1b presents in a schematic way an example of a displaceable panel of the mobile X-ray unit. In this enlarged view 10a specific elements of the displaceable panel 5 are depicted. Accordingly, a handle 6 may be implemented as a mechanical item for pulling or pushing the panel 5. Alternatively, the handle 6 may be arranged as an electrical actuator for triggering motors (not shown) for displacing the panel 5. For example, when the handle 6 is pulled the motors may be activated for causing the panel 5 to displace in direction A. Pushing of the handle 6 may cause lowering of the panel 5 in direction B. Preferably, the mobile X-ray unit comprises means for limiting the movement of the panel 5. This may be advantageous for ensuring mechanical stability of the system on one hand (limitation of the upper level) and, on the other hand, may be beneficial for preventing cable damage (limitation of the lower level). Preferably, the panel 5 is movable using built-in rails whose length may be chosen for limiting the displacement range of the panel 5 in a desirable way.

The base 2 preferably further comprises a display 7, which may function as a suitable user interface 7a. For example, the patient data, such as a photo of the patient and/or a photo of a lesion may be provided in window 7b, whereby relevant patient information, such as the date of birth, gender, dose prescription and dose delivery protocol and so on may be displayed in window 7c. Buttons 7d may be provided as touch functionality for enabling entering data. Alternatively or additionally, suitable hardware switches or buttons may be provided as well.

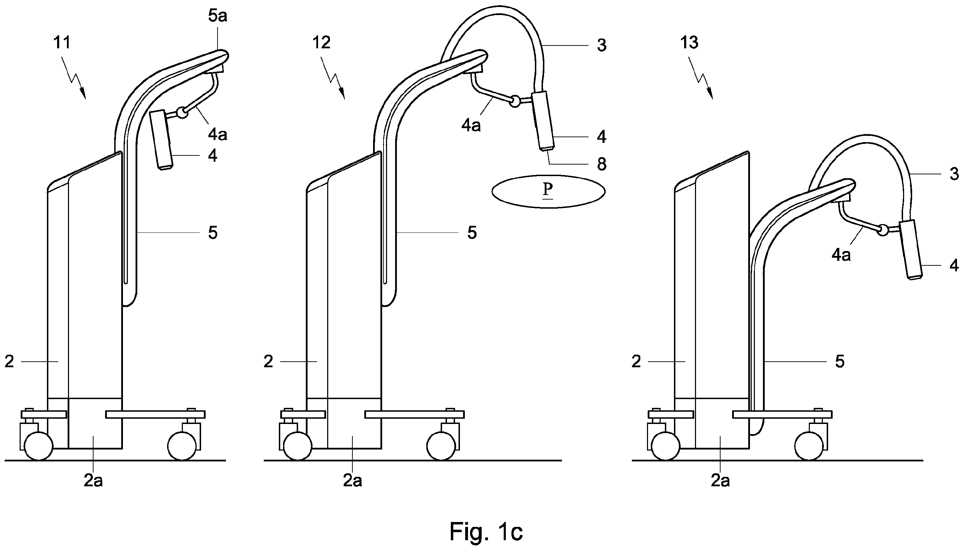

Figure 1c present in a schematic way an example of displacement functionality of the applicator of the X-ray unit. Mechanics of the mobile X-ray unit may be developed and realized to support a broad range of translational and rotational movements for the X-ray applicator 4.

In view 11 a schematic example is presented wherein the X-ray applicator is in its parked position. It will be appreciated that cabling is not depicted for clarity reasons. Such position may be suitable for transport of the mobile X-ray unit towards a booth and/or for maneuvering the X-ray unit around the patient. In order to retract the X-ray applicator as close as possible to the base 2, the articulated arm 4a may be bent under the outer portion 5a of the displaceable panel 5. For ensuring stability of the mobile X-ray unit during maneuvering thereof, a load block 2a close to a floor is provided for lowering an absolute position of the point of gravity of the overall construction.

View 12 presents in a schematic way a further possibility, wherein the X-ray application 4 is in one of its working positions having an X-ray exit surface 8 being oriented towards a patient P. In order to suitably position the X-ray applicator with respect to the patient P, the displaceable panel may be moved to a certain dwell position located between the lowest position and the highest position of the panel 5. The articulated arm 4a may be used for suitably rotating the X-ray applicator about a rotation axis. Preferably, a rotation axis is selected to coincide with a direction of emanation of the X-ray beam from the exit surface for a vertically oriented X-ray applicator.

View 13 presents in a schematic way a still further possibility, wherein the X-ray applicator 4 is to be used at a lowered position. For this purpose the displaceable panel 5 may resume its lowest stand and the arm 4a may be used for orienting the X-ray applicator in a desirable way.

The base of the mobile X-ray unit may be supported by a suitable set of wheels supported by a frame. Preferably, the wheels are interconnected by a deformable frame which ensures that all wheels make contact with an underlying surface, such as a floor or ground, even if such surface is not completely flat.

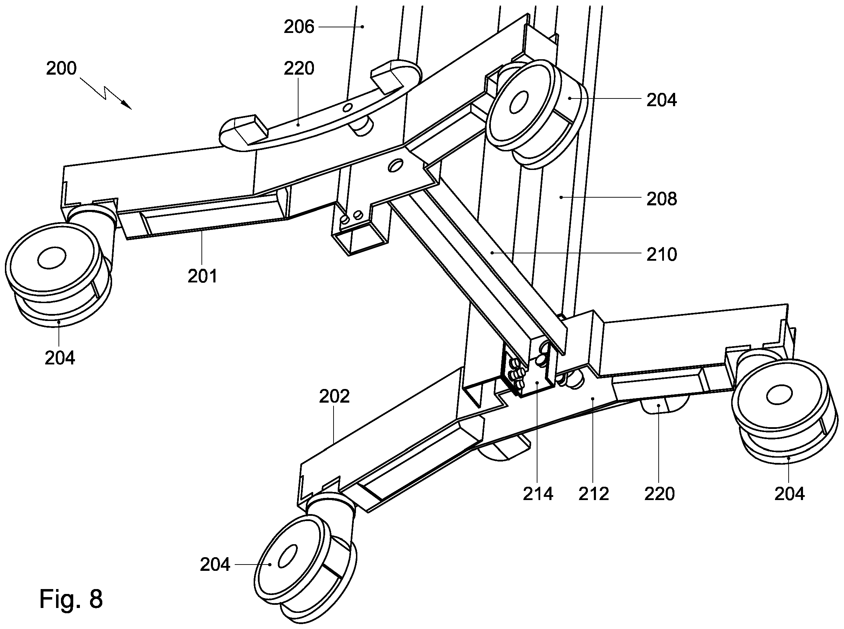

For example, the frame may comprise one or more branches working together or individually for supporting the wheels of the base. When the weight of the mobile X-ray unit is applied the branch shall be deformed allowing the full contact of all of the wheels with the ground. The frame may comprise flexible regions, adapted to be resilient and/or bendable under application of the weight of the mobile X-ray unit. A spring or other resilient item, such as a rubber, may be used for implementing the flexible regions of the frame. The medical care unit comprising the flexible frame is discussed with reference to Figures 8 - 10.

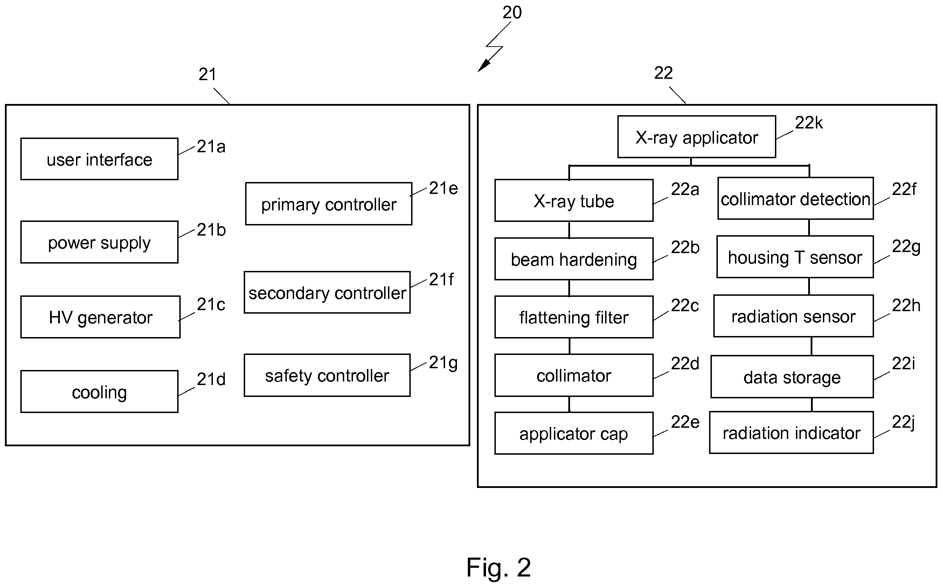

Figure 2 presents in a schematic way an example of architecture of the mobile X-ray unit. The mobile X-ray unit comprises a high voltage supply, preferably adapted to generate 50 - 75 kV X-rays in a suitable X-ray tube, a cooling system for cooling the X-ray tube during use and a control system for controlling electronic and electric parameters of sub-units of the X-ray unit during use. View 20 schematically depicts main units of the control system 21 and of the X-ray applicator 22.

The control system 21 preferably comprises a hard wired user interface 21a for enabling switching on and switching off of the high voltage supply 21b. Preferably, the high voltage supply 21b comprises a high voltage generator 21c with improved ramp-up and ramp-down characteristics. The high voltage supply is preferably operable for delivering power of about 200 W in use. Preferably, the ramp-up time is of the order of 100 ms. The hard wired interface 21a, may also be arranged to automatically switch on the cooling system 21d when the high voltage generator is switched on. In addition, the control system 21 may comprise a primary controller 21e arranged for controlling the dose delivery from the X-ray applicator in use. Such primary controller 21e may be provided with a primary counter adapted to register time lapsed after the X-ray radiation is initiated. The primary counter may then automatically switch off the high voltage supply to the X-ray tube in the event a pre-determined dose is reached. It will be appreciated that the pre-determined dose is at least dependent on the energy of generated X-rays and the dose rate, wherein such dependence may be calibrated in advance. Provided corresponding calibrated data is made available to the primary controller adequate primary dose delivery control may be achieved. Preferably, a secondary controller 21f is provided for enabling an independent loop of dose delivery control. The secondary controller may be connected to a dose meter accommodated inside the X-ray applicator in the X-ray field before the collimator. Accordingly, the dose meter may provide real-time data on actual dose delivery taking into account dose variation during ramp up and ramp down of the high voltage source. Still preferably, the control system may further comprise a safety controller 21g adapted to compare readings from the primary controller 21e and the secondary controller 21g for triggering switching off of the high voltage generator 21c wherein a desired dose is delivered. In addition or alternatively, the safety controller 21g may be wired to guard emergency stop, door interlock and a generator interlock.

The X-ray applicator 22 may preferably comprise the following features: an X-ray tube 22a, conceived to be housed in an outer housing (shielding) 22k. The X-ray tube may be provided having a target-collimator distance of about 4 - 10 cm, preferably about 5 to 6 cm. The X-ray applicator may further comprise a beam hardening filter 22b selected to intercept low-energy radiation and a beam flattening filter 22c, designed to intercept portions of X-ray radiation for generating a substantially flat beam profile near the exit surface of the X-ray applicator. Further, the X-ray applicator 22 may comprise one or more collimators arranged to define treatment beam geometry. Preferably a set of collimators is used, for example, having diameters of 1, 1.5, 2, 2.5, 3, 3.5, 4, 4.5, 5 cm. It will be appreciated that although circular collimators are discussed, collimators of any shape, such as square, elliptic or custom made collimators are possible. It is found to be advantageous to provide the X-ray applicator 22 with automatic collimator detection means 22f adapted to automatically signal which collimator is being used. Preferably, resistive sensing is used, wherein each collimator is provided with at least a couple of projections for bridging a resistive path provided in a collimator receptacle. The resulting electrical resistance of the receptacle constitutes a signal representative of a collimator being used. The X-ray applicator 22 still further preferably comprises a built-in temperature sensor adapted to signal temperature of the X-ray tube and/or its shielding. The signal from the temperature sensor is received by the control system which carried out analysis thereof. Should the measured temperature be elevated beyond an allowable level, an alarm signal may be generated. Optionally, a shut-off signal to the high voltage generator may be provided. The X-ray applicator 22 further comprises a radiation sensor 22h arranged inside the outer housing 22k for detecting X-ray radiation which is actually being delivered by the X-ray tube. Preferably, for safety reasons the X-ray applicator 22 further comprises a non-volatile data storage 22i arranged for recording operational parameters at least of the X-ray tube. Further, to enhance radiation safety, the X-ray applicator 22 may be provided with a radiation indicator 22j arranged for providing a visual and/or an audio output to the user and/or the patient regarding ON/OFF condition of the X-ray tube. It will be appreciated that the radiation indicator 22j may comprise a plurality of distributed signaling means. Preferably, at least one signaling means, for example a light emitting diode (LED) is associated with the X-ray applicator 22. More preferably, the signaling means is provided on the X-ray applicator 22.

Figure 3 presents in a schematic way an example of a cross section of an X-ray applicator of the mobile X-ray unit. The X-ray applicator 30 comprises an outer housing 36 accommodating the X-ray tube assembly 35 provided with external shielding 35a. In use the X-ray applicator may be maneuvered by the user by holding the housing 32. The X-ray applicator 30 further comprises a target 45 arranged to emit a beam of X-rays having a longitudinal propagation axis 45a. The distance between the target (anode) and the collimator 33 may be in the range of 4 ... 10 cm, preferably about 5 to 6 cm. Such relatively short target-collimator distance is surprisingly suitable for generating an X-ray beam having a substantially narrow penumbra (1.5 - 1.8 mm for 20/80% lines) and good beam flatness.

The X-ray applicator 30 further comprises a filter 39 for hardening the X-ray beam emanating from the target 45, a beam flattening filter 40 for flattening out a beam profile and collimator 33 insertable in a collimator receptacle 41.

In order to prevent overheating of the X-ray tube in use a cooling system 34 is provided, which may advantageously be arranged in spacing between the X-ray tube 35 and the shielding 35a in contact with the surface of the X-ray tube 35. A suitable coolant may be provided using a pipe 31. Preferably, the coolant is circulating and may be water, a pressurized gas or even a special oil. The X-ray applicator may further comprise a temperature sensor 37.

The X-ray assembly 30 may further comprise a suitable radiation detector 38, connected to a radiation indicator 43. Preferably, data collected by the radiation detector 38 is stored in a data storage unit 44. In order to protect an X-ray exit surface of the X-ray applicator 30 from intra-patient contamination, an applicator cap 42 may be provided to cover at least the exit surface of the X-ray applicator 30. Preferably, the applicator cap is thick enough to fully intercept secondary electrons emanating from the X-ray applicator. Preferably, the applicator cap is manufactured from PVDF (polyvinylidene fluoride) and is about 0.4 - 0.7mm, preferably 0.6 mm thick across the window portion, having density of about 1.75 - 1.8, preferably 1.78. Alternatively the applicator cap may be 0.3 - 0.6mm, preferably 0.5 mm thick across the window portion and having density of 1.30 - 1.45, preferably 1.39, being manufactured from PPSU (polyphenylsulfone). It is found that these materials are particularly suitable as they as stable under influence of the X-rays and are suitable for different types of sterilization procedures, such as chemical sterilization, or sterilization under elevated temperatures.

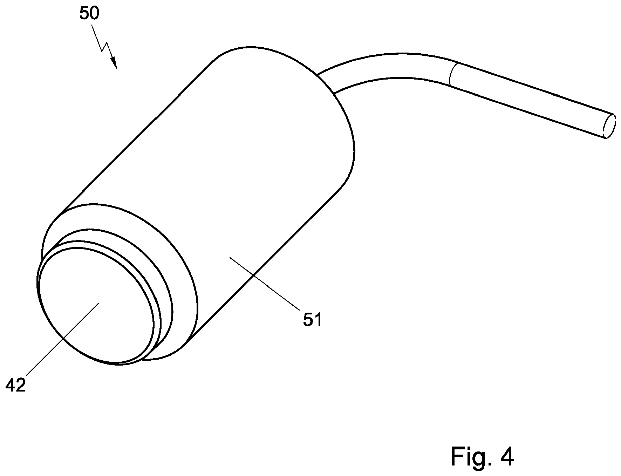

Figure 4 presents in a schematic way an example of the X-ray applicator of Figure 3 provided with an applicator cap. The applicator cap 42 may be manufactured from transparent glass, transparent plastic or from ceramics as well as from PVDF and PPSU as is set forth in the foregoing. It is also possible, although not preferable to manufacture the applicator cap from a metal. In the latter case the applicator cap may be sterilized, however, it is preferable to use a disposable applicator cap. In view 50 of Figure 4 it is seen that the outer dimension of the X-ray applicator 51 may be larger that the outer dimension of the exit portion covered by the applicator cap 42. Although such configuration is preferable for minimizing total weight of the X-ray applicator, it is possible that the exit portion has the same dimension as the body of the X-ray applicator 51.

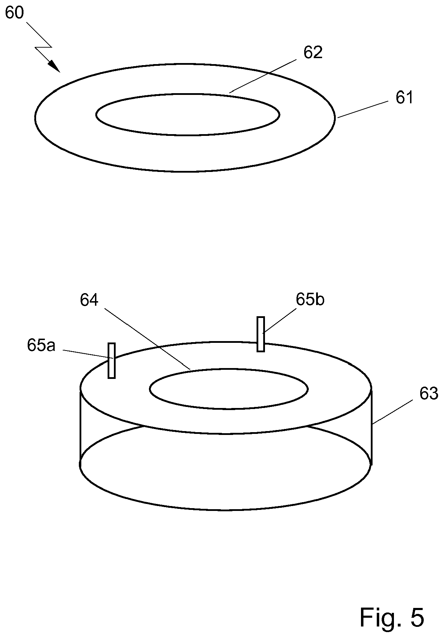

Figure 5 presents in a schematic way an example of a collimator provided with identification means. The collimator 63 is provided with a central opening 64 for defining a shape and dimension of the resulting X-ray beam emanating from the X-ray applicator 30 as is discussed with reference to Figure 3. The collimator 63 is adapted to be received in a collimator receptacle 61, which may be shaped as a suitable chamber wherein the collimator 63 is to be firmly fitted. In order to enable automatic collimator identification, the collimator is provided with two projections 65a, 65b adapted to interact with a resistive path 62 provided in the collimator receptacle 61. When the projections 65a, 65b come into contact with the path 62 a net resistance of the collimator receptacle will be changed. The change in the resistance of the collimator receptacle is used as an automatic identifier of the collimator being inserted in the collimator receptacle. It will be appreciated that for a set of collimators, each collimator has to be provided with a unique pair of projections leading to a distinguishable change in the net resistivity of the collimator receptacle. Those skilled in the art will readily appreciate that a plurality of pairs 65a, 65b having different respective positions on a surface of the collimator may be envisaged. Alternatively, it is possible to provide each collimator with electronic identification means, for example, a chip cooperating with a plug. When the plug is plugged-in the collimator receptacle (provided with a cooperating socket) the collimator identification may be communicated to the control unit of the mobile X-ray unit.

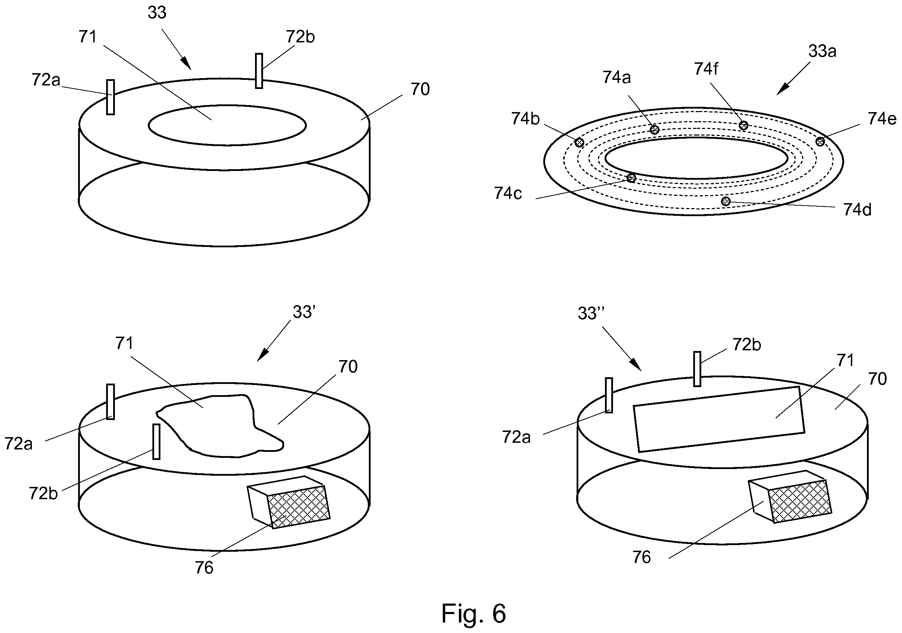

Figure 6 presents in a schematic way an alternative example of a collimator provided with identification means. Different embodiments of a collimator 33, shown in Figure 3, will be discussed here in more detail. The collimator 33 may be provided with an aperture 71, which may have any shape. The identification means 72a, 72b may be used for automatically detecting whether a correct (i.e. intended) collimator is being inserted in the X-ray applicator. For example, the identification means 72a, 72b may refer to suitable spring loaded pins arranged for interacting with a resistive body (shown in the view 33a) for causing a change in a net resistance of the resistive body. By detecting a signal representative of the absolute or relative resistance of the resistive body an automatic identification of the inserted collimator may be carried out.

In view 33a a schematic example of the resistive body is depicted, wherein each dot of the series 74a, 74b, 74c, 74d, 74e, 74f is attributed to a separate resistive contact circle (only few are shown for clarity). The net resistive change of the resistive path 33a depends upon where the pin 72a or 72b contacts a resistive circle of the resistive circuit 33a and will change according to the contact positions. The individual collimators of the type 33, may be coded by differently positioning the contact pins 72a, 72b on the outer surface 70.

In alternative examples 33', 33", the contact pins 72a, 72b may be supplemented by a contact bar 76, used for locking and/or enabling an appropriate insertion of the collimator into a collimator receptacle. This feature is particularly advantageous for collimators not having rotational symmetry.
In a still further examples, the collimators and/or the pins may be color coded.

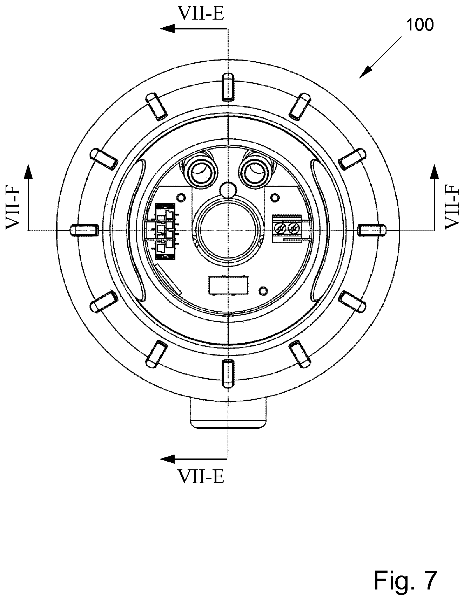

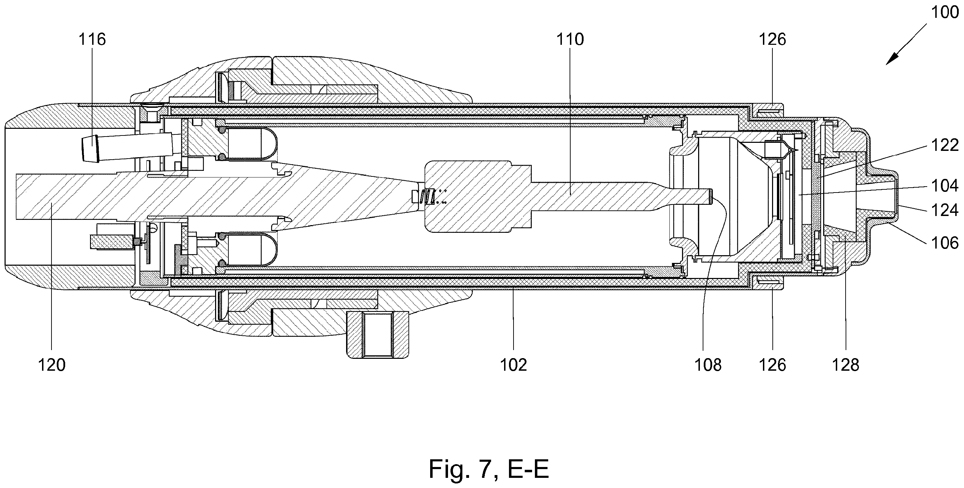

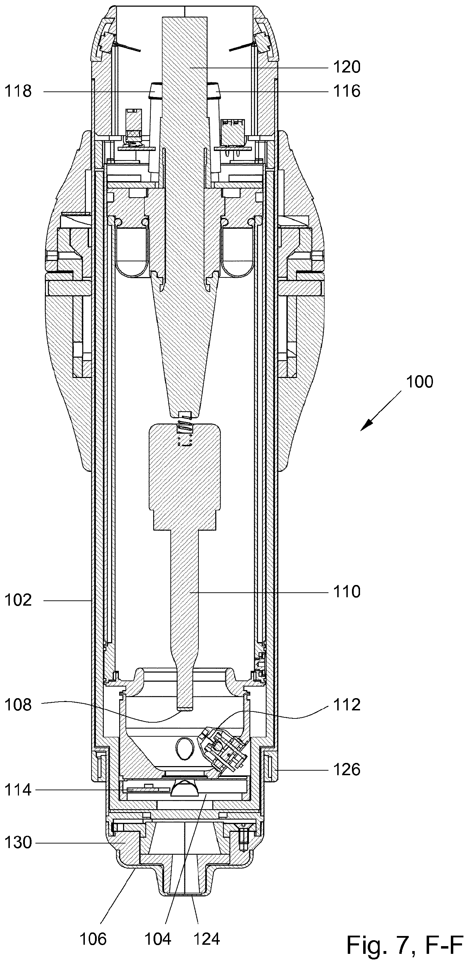

Figure 7 describes in a schematic way a further example of the X-ray tube. The X-ray tube 100, has a body 102 enclosing at one end an end window 104 through which the X-rays pass, see Figure 7 cross-section E-E. The end window is made from a thin sheet of Beryllium metal. Covering the end window 104 to provide protection against the damage of the window and protection against the toxic effects of the metal is an applicator cap 106. Applicator cap 106 is preferably made from a plastic material. Preferably, the applicator cap is manufactured from PVDF (polyvinylidene fluoride) and is about 0.4 - 0.7mm, preferably 0.6 mm thick across the window portion, as described in more detail above, or alternatively the applicator cap may be manufactured from PPSU (polyphenylsulfone) and be 0.3 - 0.6mm, preferably 0.5 mm thick across the window portion, also as described above in more detail.

In the tube body 102 a target 108 is located at between 4 -10 cm from the collimator 130, and preferably at 4-6cm from the collimator 130 (see Figure 7, cross-section F-F). It will be appreciated that this distance is measured between the outer surface of the target 108 and a midplane of the collimator 130. The target is made from Tungsten metal to provide the desired X-ray spectrum. The tungsten tip of the target is mounted on a large anode assembly 110 which also serves to conduct away the heat created from the generation of the X-rays in the target. Most of the anode assembly is made from copper. The cathode 112 (see Figure 7, cross-section F-F) is located slightly off-axis near the end window. Electrons emitted from the cathode are accelerated across the gap by the potential difference between the cathode and anode, in this case set at about 70kV, to the target which they impact and cause the generation of X-rays in a known manner. X-rays emitted from the target 108 pass through a beam hardening filter 122 before passing through a collimator 130 and an exit surface 124 on an applicator cap 106. The collimator 130 may be housed in a suitable collimator receptacle 128. The anode assembly 110 is mounted in the body 102 and electrically insulated from it. One of a number of known techniques and materials can be used to provide the desired level of insulation between the anode and the body 102.

As is also well known in the art, the production of X-rays generates large amounts of waste heat, with the result that it is necessary to cool the tube in order to maintain it at a safe temperature. Various cooling mechanisms are known and used in the art. In this example, the X-ray tube is cooled by means of cooled water forced around the anode region. Cooled water enters the back of the tube by means of conduits 116 and leaves by means of a second conduit 118 (see Figure 7, cross-section F-F). The water cooling circuit is a closed loop circuit, with the water leaving the tube assembly to be cooled by a remote cooler (not shown) before returning to the tube. Alternatively oil or another liquid could be used as the cooling medium. It is also known that a pressurized gas is used as an effective coolant in some applications.

As is known in the art, X-rays are generated and emitted in all directions, but the shielding by the body of the tube 102 and other internal components will tend to reduce the amount of radiation emitted from the body of the tube to a minimum, with most of the radiation emitted from the end window. The thickness of the shielding provided by the body is designed such that it provides at least the minimum level of shielding required for safe use by the operator.

A high voltage cable assembly 120 is connected to the anode assembly 110. The high voltage cable assembly is connected to flexible cable means (not shown) which in turn is connected to a high voltage power supply.

A radiation detector 114 is placed outside the path of the X-ray beam emitted from the target 108 and passing through the end window 104. This detector can be any known form of radiation detector. In this embodiment it is a known form of suitably radiation hardened semi-conductor connected to an amplifier. The radiation detector 114 detects when the tube 102 is working and emitting X-ray energy. Output from the detector is connected to a control unit, the output signals from which may be used to provide an optical indication to a user of whether the tube is operating or not. By this means an X-ray detector is provided which can be used to detect whether the tube is on or off.

In order to enable the tube 102 to be placed accurately over a tumour, a tumour illumination means is used. The tumour illumination means comprises a plurality of lights 126 placed around the circumference of the tube near the end window. When in use, the lights shine onto the skin of the patient. Since the lights 126 are positioned around the circumference of the tube body 102, at a short distance from the end of the tube they create a circle of light with a sharp cut off of the inner part of the circle. In this way, the position of the lights on the tube body 102 creates a shadow. This shadow circle is used to indicate the region which will be subject to irradiation when the X-ray tube is turned on. It should be appreciated the area within the circle will not be completely dark; the ambient light will be able to enter the shadow region.

Preferably the lights 126 are white LEDs which can be bright enough to clearly illuminate the target region but do not generate amounts of heat and have very long lives. The lack of heat generation is important because the lights will be in close proximity to the skin of the patient, and so it is important to minimise the risk of burning or other damage to the skin. Other colours of LEDs could be used. Alternatively, other light sources could be used, such as known filament lamps or even a remote light source connected to the ring by fibre optic cables.

With further calibration of the radiation detector 114, it is possible to determine and calculate the X-ray dose administered to the patient during the treatment. By this means it is possible to have a real time dosimetry measurement system, in which the precise amount of radiation dose administered can be determined. Once the dose rate is known, a treatment plan can be modified during treatment. This is advantageous because it enables a very accurate and carefully controlled dose of X-rays to be administered.

Figure 8 presents in a schematic way an example of a medical care unit, such as a mobile X-ray unit. The mobile X-ray unit may be constructed on a rolling chassis 200. The chassis may be in the shape of an H section when viewed in plan. Preferably, the legs of the H section are splayed and extend slightly outwards to provide increased stability. The chassis may have four wheels 204 which may be independently rotatable and castoring and can be used to manoeuvre the mobile X-ray unit into a desired position.

The chassis may also be provided with a braking mechanism, which may be operated by a pedal. Twin pedals 220 may be provided, one on each side of the chassis. The pedals are preferably connected by a shaft, thereby ensuring that only one pedal needs to be operated to brake the chassis against movement. The braking mechanism may be arranged to brake diametrically opposed wheels. Other braking mechanisms can be envisaged as well.

The chassis legs 201, 202 in the present example are in the form of strong structural members, such as pressed metal channels or beams. The two legs 201and 202 are joined by a cross-member 210. The cross member 210 may be of the C shaped cross -section and may be secured at or near its ends to the legs 201 and 202 by per se known means, such as bolts or welding.

In the present example, the legs and cross-member are made from pressed metal parts but many other suitable shapes and materials can be envisaged. It can be envisaged that the rolling part of the chassis could also be formed from a molded plastics material or in cases requiring higher strength, load carrying characteristics or rigidity they could be made from cast metal structures.

A first vertical chassis member 206 may be securely attached to a first of the legs 201 and may extend upwardly there from. Connected to the second leg 202 is a second vertically extending chassis member 208. Vertical chassis members 206 and 208 are securely connected together by known means not shown. The operational equipment forming the mobile X-ray unit, for example the high voltage power supply, the cooling system for the X-ray tube and the control system, may be mounted on the vertical chassis members 206, 208. Also mounted on the vertical members is the moveable arm (not shown). This configuration has an advantage that the vertical chassis members do not need to be vertical but can be upwardly extending at any angle that is convenient and appropriate for the mounting of any ancillary fittings or equipment.

The first chassis leg 201 is firmly connected to the vertical chassis member 206 by means of bolts, which facilitate assembly of the chassis. However, other known means of securely fixing two components together, such as by welds can be used. The second vertical chassis member 208 is connected to the second leg 202 by means of a bearing structure. Second vertical chassis member 208 is firmly secured to a mounting bracket 214 by means of bolts, welds or any other known securing means. The mounting bracket is provided with a bearing support means which co-operates with corresponding bearing means in the second leg 202. The bearing is conveniently in the form of a shaft or pin 212. Shaft 212 extends through a bearing support means in the mounting bracket 214 into a co-operating support means in the leg 202. The shaft and co-operating bearing holes enable the second vertical member 208 to rotate about an axis defined as extending along a longitudinal axis extending along the length of the shaft 212. The bearing support means may be made of any known form of bearing material, such as a relatively soft metal such as brass or preferably from a nylon or polyethylene type plastics material.

In operation the vertical chassis members 206, 208 are firmly connected together by means not shown here to provide a strong rigid upwardly extending chassis onto which any other components required can be mounted, whilst the rolling part of the chassis is provided with a flexibility to enable it to accommodate rough or uneven surfaces.

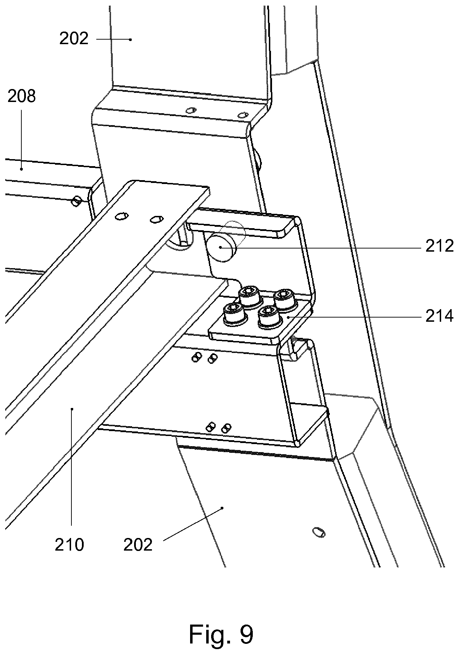

Figure 9 presents a further view of a connection between the various components of the flexible frame. It will be appreciated that the construction details for the vertical chassis member 206 may be similar. The bearing comprising the shaft 212 defined to have a rotational axis passing through the centre of the shaft 212 about which the leg 202 can rotate, so providing a means of allowing the rolling part of the chassis to deform and adapt to uneven floors or paths whilst maintaining a relatively stiff upwardly extending chassis portion. The shaft 212 extends through a bearing support means in the mounting bracket 214 into a co-operating support means in the leg 202 (shown in Figure 8). This construction allows the legs 201, 202 (shown in Figure 8) to rotate with respect to one another as they move over (or rest on) uneven surfaces, so reducing the risk of instability of the equipment as a whole.

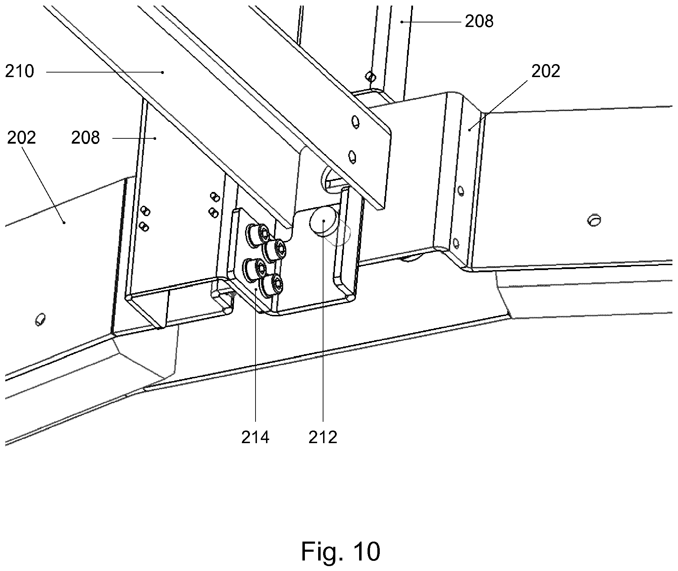

Figure 10 presents a further schematic view of an example shown in Figure 9. The leg 202 is thus mechanically linked by bearing means 212 through the mounting bracket 214 to the chassis member 208. As described above, the cross member 210 is attached at or near each of its ends to one of the legs 201, 202. It in effect provides the means to keep the legs in their chosen relative positions when the unit is stationary. However, it will be subject to rotational twisting and torque as the chassis moves over uneven ground. The structural strength of the cross member will generate forces to resist the twist of the legs with respect to one another and so will also provide a damping effect to restrict and cushion the relative movement of the legs. It will be apparent that the rotational stiffness of the cross member can be chosen to provide the desired damping effect taking into consideration the weight of the mobile unit and the un-evenness of the ground being traversed.

While specific embodiments and example have been described above, it will be appreciated that the invention may be practiced otherwise than as described. The descriptions above are intended to be illustrative, not limiting. Thus, it will be apparent to one skilled in the art that modifications may be made to the invention as described in the foregoing without departing from the scope of the claims set out below.

## Claims

1. A mobile X-ray unit (10) comprising a base (2) for accommodating a control unit, a power supply and a cooler and further comprising an articulated displaceable arm (4a) supporting an X-ray applicator (4, 22, 30, 51) provided with an X-ray tube (100), said X-ray applicator (4) being connected to the base, the X-ray tube comprising a target for generating an X-ray beam and a collimator (130) for shaping the generated X-ray beam,
wherein the X-ray applicator (4) comprises an exit surface (8) conceived to be directed towards a patient, the X-ray applicator (4) further comprising an applicator cap (106) for covering at least said surface and for intercepting secondary electrons emanating from the X-ray applicator, **characterized in that** the applicator cap (106):
is manufactured from polyphenylsulfone having a thickness between 0.3 - 0.6 mm, or
is manufactured from polyvinylidene fluoride having a thickness between 0.4 - 0.7 mm .

2. The mobile X-ray unit according to claim 1, wherein the applicator cap is disposable.

3. The mobile X-ray unit according to claim 1 or 2, wherein thickness of the cap in a direction of the beam propagation is sufficient for substantially eliminating electron contamination from the X-ray beam.

4. The mobile X-ray unit according to any of the preceding claims,
wherein the applicator cap functions as a heat absorber for mitigating the elevated temperature of the X-ray applicator in use.

5. The mobile X-ray unit according to claim 1, wherein the target and the collimator are accommodated in a substantially cylindrically shaped X-ray tube having a longitudinal axis, a direction of propagation of the X-ray beam being substantially parallel to said longitudinal axis.

6. The mobile X-ray unit according to any of the preceding claims,
wherein a distance between the target and the collimator is in the range between 4 and 10 cm.

7. The mobile X-ray unit according to any one of the preceding claims, further comprising a temperature sensor for measuring actual temperature of an outer surface of the X-ray applicator.

## Patentansprüche

1. Mobiles Röntgengerät (10), umfassend eine Basis (2) zum Aufnehmen einer Steuereinheit, einer Stromversorgung und eines Kühlers, und ferner umfassend einen gelenkig verschiebbaren Arm (4a), der einen Röntgenapplikator (4, 22, 30, 51), versehen mit einer Röntgenröhre (100) trägt, welcher Röntgenapplikator (4) mit der Basis verbunden ist, die Röntgenröhre umfassend ein Ziel zum Erzeugen eines Röntgenstrahls und einen Kollimator (130) zum Bilden des erzeugten Röntgenstrahls,
wobei der Röntgenapplikator (4) eine Ausgangsoberfläche (8) umfasst, konzipiert um zu einem Patienten gerichtet zu werden, der Röntgenapplikator (4) ferner umfassend eine Applikatorkappe (106) zum Bedecken von mindestens der Oberfläche und zum Abfangen sekundärer Elemente, ausgestrahlt von dem Röntgenapplikator, **dadurch gekennzeichnet, dass** die Applikatorkappe (106):
hergestellt ist aus Polyphenylsulfon mit einer Dicke zwischen 0,3-0,6 mm, oder
hergestellt ist aus Polyvinylidenfluorid mit einer Dicke zwischen 0,4-0,7 mm.

2. Mobiles Röntgengerät nach Anspruch 1, wobei die Applikatorkappe wegwerfbar ist.

3. Mobiles Röntgengerät nach Anspruch 1 oder 2, wobei die Dicke der Kappe in einer Richtung der Strahlausbreitung ausreichend ist, um im Wesentlichen Elektronenkontamination von dem Röntgenstrahl zu eliminieren.

4. Mobiles Röntgengerät nach einem der vorhergehenden Ansprüche, wobei die Applikatorkappe als ein Hitzeabsorbierer zum Abschwächen der erhöhten Temperatur des Röntgenapplikators bei Verwendung funktioniert.

5. Mobiles Röntgengerät nach Anspruch 1, wobei das Ziel und der Kollimator in einer im Wesentlichen zylinderförmigen Röntgenröhre mit einer Längsachse aufgenommen sind, mit einer Ausbreitung des Röntgenstrahls im Wesentlichen parallel zu der Längsachse.

6. Mobiles Röntgengerät nach einem der vorhergehenden Ansprüche, wobei ein Abstand zwischen dem Ziel und dem Kollimator im Bereich zwischen 4 und 10 cm ist.

7. Mobiles Röntgengerät nach einem der vorhergehenden Ansprüche, ferner umfassend einen Temperatursensor zum Messen der Ist-Temperatur einer Außenoberfläche des Röntgenapplikators:

## Revendications

1. Unité à rayons X mobile (10) comprenant une base (2) pour loger une unité de commande, une alimentation électrique et un refroidisseur et comprenant en outre un bras articulé déplaçable (4a) supportant un applicateur de rayons X (4, 22, 30, 51) muni d'un tube à rayons X (100), ledit applicateur de rayons X (4) étant relié à la base, le tube à rayons X comprenant une cible pour générer un faisceau de rayons X et un collimateur (130) pour mettre en forme le faisceau de rayons X généré,
dans lequel l'applicateur de rayons X (4) comprend une surface de sortie (8) conçue pour être dirigée vers un patient, l'applicateur de rayons X (4) comprenant en outre un capuchon d'applicateur (106) pour recouvrir au moins ladite surface et pour intercepter des électrons secondaires émanant de l'applicateur de rayons X, **caractérisée en ce que** le capuchon d'applicateur (106) :
est fabriqué à partir de polyphénylsulfone ayant une épaisseur comprise entre 0,3 et 0,6 mm, ou est fabriqué à partir de polyfluorure de vinylidène ayant une épaisseur comprise entre 0,4 et 0,7 mm.

2. Unité à rayons X mobile selon la revendication 1, dans laquelle le capuchon d'applicateur est jetable.

3. Unité à rayons X mobile selon la revendication 1 ou 2, dans laquelle l'épaisseur du capuchon dans une direction de la propagation de faisceau est suffisante pour éliminer sensiblement une contamination par électrons provenant du faisceau de rayons X.

4. Unité à rayons X mobile selon l'une quelconque des revendications précédentes, dans laquelle le capuchon d'applicateur fonctionne comme un absorbeur de chaleur pour atténuer la température élevée de l'applicateur de rayons X en utilisation.

5. Unité à rayons X mobile selon la revendication 1, dans laquelle la cible et le collimateur sont logés dans un tube à rayons X de forme sensiblement cylindrique ayant un axe longitudinal, une direction de propagation du faisceau de rayons X étant sensiblement parallèle audit axe longitudinal.

6. Unité à rayons X mobile selon l'une quelconque des revendications précédentes, dans laquelle une distance entre la cible et le collimateur est comprise entre 4 et 10 cm.

7. Unité à rayons X mobile selon l'une quelconque des revendications précédentes, comprenant en outre un capteur de température pour mesurer une température réelle d'une surface extérieure de l'applicateur de rayons X.
